(19) Europäisches Patentamt
European Patent Office
Office européen des brevets

(11) **EP 4 585 159 A1**

(12) **EUROPEAN PATENT APPLICATION**

(43) Date of publication:
16.07.2025 Bulletin 2025/29

(21) Application number: 24305095.2

(22) Date of filing: 15.01.2024

(51) International Patent Classification (IPC):
*A61B 6/50* $^{(2024.01)}$     *G06T 11/00* $^{(2006.01)}$

(52) Cooperative Patent Classification (CPC):
**G06T 11/008; A61B 6/504;** G06T 2211/404

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **FLORENT, Raoul**
**5656 Eindhoven (NL)**
• **LAI, Marco**
**5656 Eindhoven (NL)**
• **HENDRIKS, Bernardus**
**5656 Eindhoven (NL)**

(74) Representative: **Philips Intellectual Property & Standards**
**High Tech Campus 52**
**5656 AG Eindhoven (NL)**

(54) **IMAGE REPRESENTATION OF A VASCULAR REGION FROM VOLUMETRIC AND ANGIOGRAPHIC IMAGE DATA**

(57) A system for providing an image representation of a vascular region, is provided. The system comprises one or more processors configured to: receive volumetric angiographic image data comprising a volumetric image (120) of the vascular region; receive projection angiographic image data comprising one or more projection images ($130_{1..i}$) of the vascular region; adapt the volumetric image (120) using the one or more projection images ($130_{1..i}$) to provide an adapted volumetric image ($120^A$); and output an image representation of the vascular region, the image representation being generated using the adapted volumetric image ($120^A$).

FIG. 3

**Description**

TECHNICAL FIELD

The present disclosure relates to providing an image representation of a vascular region. A system, a computer-implemented method, a computer program product, and a projection angiographic imaging system, are disclosed.

BACKGROUND

Vascular conditions are often diagnosed using volumetric angiographic images, i.e. three-dimensional angiographic images. Imaging modalities such as computed tomography "CT", magnetic resonance imaging "MRI", and three-dimensional "3D" ultrasound, are typically used to acquire such images. If an interventional procedure is deemed necessary, the volumetric angiographic image is often used to plan the procedure. For instance, a volumetric angiographic image of a vascular region such as the heart is often used to plan a path to a target location in which an intervention is to be performed, or to determine the size of a stent to use at the target location. Interventional procedures, by contrast, are typically performed using projection angiographic images, i.e. two-dimensional angiographic images. The projection angiographic images are typically X-ray projection angiographic images that are generated by a projection X-ray imaging system. During the interventional procedure, a physician acquires such projection angiographic images of the vascular region. The physician uses the projection angiographic images to perform tasks such as the navigation of interventional devices through the vasculature, and stent deployment, whilst referring-back to the planned path in the volumetric angiographic image.

The process of referring-back to a volumetric angiographic image during an interventional is cumbersome, however. Factors such as the tortuous, overlapping, bifurcating, nature of vessels in the vasculature, and the typically inferior image resolution in the volumetric angiographic image, hamper the physician's ability to visualize the correspondence between the projection angiographic images and the volumetric angiographic image. Moreover, the vessels in a vascular region often move, and deform over time as a result of cardiac motion, breathing, patient re-positioning, and also the presence of interventional devices in the vasculature. Such movements and deformations of the vessels, further hamper the physician's ability to visualize the correspondence between the projection angiographic images and the volumetric angiographic image.

Thus, there remains a need to improve the visualization of vascular regions.

SUMMARY

According to one aspect of the present disclosure, a system for providing an image representation of a vascular region, is provided. The system comprises one or more processors configured to:

receive volumetric angiographic image data comprising a volumetric image of the vascular region;
receive projection angiographic image data comprising one or more projection images of the vascular region;
adapt the volumetric image using the one or more projection images to provide an adapted volumetric image; and
output an image representation of the vascular region, the image representation being generated using the adapted volumetric image.

The system outputs an image representation of the vascular region. The image representation is generated using an adapted volumetric image that is provided by adapting a volumetric image of the vascular region using the one or more projection images of the vascular region. The image representation therefore benefits from features that are present in the projection image(s) as well as features that are present in the volumetric image. In so doing, the system provides an improved visualization of the vascular region.

Further aspects, features, and advantages of the present disclosure will become apparent from the following description of examples, which is made with reference to the accompanying drawings.

BRIEF DESCRIPTION OF THE DRAWINGS

Fig. 1 is a schematic diagram illustrating an example of a system 100 for providing an image representation of a vascular region, in accordance with some aspects of the present disclosure.
Fig. 2 is a flowchart illustrating an example of a computer-implemented method of providing an image representation of a vascular region, in accordance with some aspects of the present disclosure.

Fig. 3 is a schematic diagram illustrating an example of the evaluation of a correspondence between the vessels $150_{1..j}$ in a plurality of projection images $130_{1..i}$ and a corresponding projection of the vessels $150^P_{1..j}$ in a volumetric image 120, in accordance with some aspects of the present disclosure.

Fig. 4 is a schematic diagram illustrating a first example of the deforming of a cross sectional shape 160 of a vessel $150_1$ in a volumetric image, in accordance with some aspects of the present disclosure.

Fig. 5 is a schematic diagram illustrating a second example of the deforming of a cross sectional shape 160 of a vessel $150_1$ in a volumetric image, in accordance with some aspects of the present disclosure.

Fig. 6 is a schematic diagram illustrating an example of the adjusting of an intensity 190 within a cross sectional shape 160 of a vessel $150_1$ in a volumetric image, in accordance with some aspects of the present disclosure.

Fig. 7 is a schematic diagram illustrating a first example of the calculated values of some example vessel parameters 210, in accordance with some aspects of the present disclosure.

Fig. 8 is a schematic diagram illustrating a second example of the calculated values of some example vessel parameters, in accordance with some aspects of the present disclosure.

Fig. 9 is an example of an image representation 140 of a vascular region including an indication of the origin of the calculated value of a vessel parameter, in accordance with some aspects of the present disclosure.

Fig. 10 is a schematic diagram illustrating an example of an image representation 140 of a vascular region including an indication of a recommended position for a stent, in accordance with some aspects of the present disclosure.

## DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, and in a computer program product, and in a projection angiographic imaging system, in a corresponding manner.

In the following description, reference is made to examples of a system for providing an image representation of a vascular region. In some examples, the vascular region is a portion of the heart. It is, however, to be appreciated that the heart serves only as an example of a vascular region. In general, the vascular region may be any anatomical region. For instance, the vascular region may alternatively be a portion of the brain, the lung, the leg, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray™ and DVD.

It is also noted that some operations that are performed by the one or more processors of the system disclosed herein may be implemented using artificial intelligence techniques. Suitable techniques may include machine learning techniques, as well as deep learning techniques such as neural networks. For instance, one or more neural networks, may be trained in a supervised, or in some cases unsupervised, manner, to implement the operations that are performed by the one or more processors.

As mentioned above, there remains a need to improve the visualization of vascular regions.

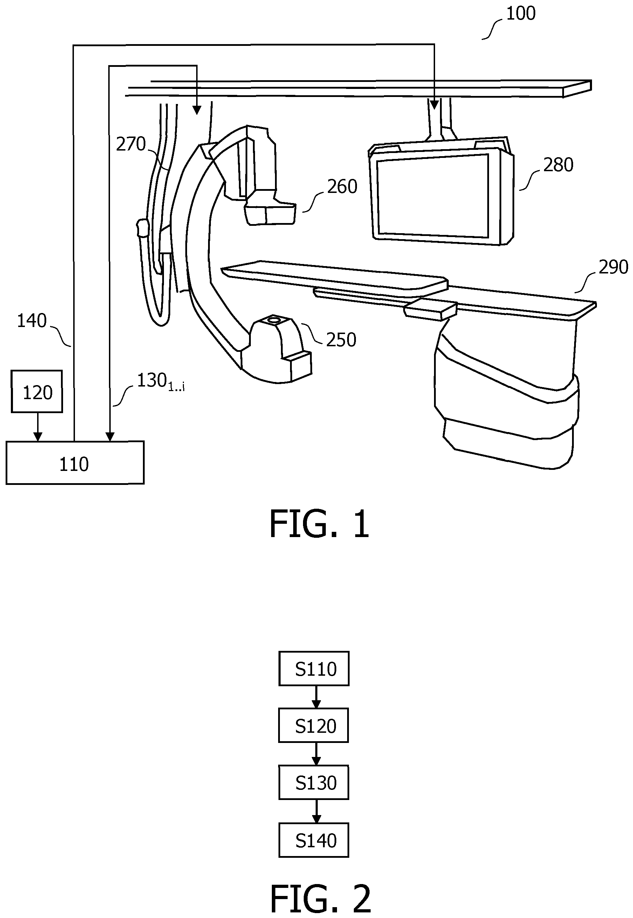

Fig. 1 is a schematic diagram illustrating an example of a system 100 for providing an image representation of a vascular region, in accordance with some aspects of the present disclosure. Fig. 2 is a flowchart illustrating an example of a computer-implemented method of providing an image representation of a vascular region, in accordance with some aspects of the present disclosure. It is noted that operations that are described as being performed by the one or more processors 110 of the system 100 illustrated in Fig. 1, may also be performed in the method illustrated in Fig. 2. Likewise, operations that are described in relation to the method described with reference to Fig. 2 may also be performed by the one or more processors 110 of the system 100 illustrated in Fig. 1. With reference to Fig. 1, and Fig. 2, the system 100 for providing an image representation of a vascular region comprises one or more processors 110 configured to:

> receive S110 volumetric angiographic image data comprising a volumetric image 120 of the vascular region;
> receive S120 projection angiographic image data comprising one or more projection images $130_{1..i}$ of the vascular region;
> adapt S130 the volumetric image 120 using the one or more projection images $130_{1..i}$ to provide an adapted volumetric image $120^A$; and
> output S140 an image representation 140 of the vascular region, the image representation being generated using the adapted volumetric image $120^A$.

The system 100 outputs an image representation of the vascular region. The image representation is generated using an adapted volumetric image that is provided by adapting a volumetric image of the vascular region using the one or more projection images of the vascular region. The image representation therefore benefits from features that are present in the projection image(s) as well as features that are present in the volumetric image. In so doing, the system provides an improved visualization of the vascular region.

The operations performed by the processor(s) of the system 100 are described in more detail below.

In the operation S110, volumetric angiographic image data is received. The volumetric angiographic image data comprises a volumetric image 120 of the vascular region. The volumetric image 120 may represent various vascular regions. For example, the volumetric image 120 may represent a portion of the heart, or a portion of another anatomical region such as the brain, the lung, the leg, and so forth. The vascular region may include one or more vessels. In general, the vessels may be arteries, veins, capillaries, and so forth.

The volumetric angiographic image data that is received in the operation S110 may be generated by various types of volumetric imaging systems. These include computed tomography "CT" imaging systems, magnetic resonance imaging "MRI" systems, and 3D ultrasound imaging systems. The CT imaging system may be a spectral CT imaging system. Spectral CT imaging systems generate X-ray attenuation data representing X-ray attenuation within multiple different energy intervals. The X-ray attenuation data generated by a spectral CT imaging system may be processed, e.g. using various material decomposition algorithms, in order to distinguish between media that have similar X-ray attenuation values when measured within a single energy interval, and which would be indistinguishable in X-ray attenuation data obtained using a conventional CT imaging system. Thus, X-ray attenuation data generated by spectral CT imaging systems may be processed to provide a volumetric angiographic image with improved specificity to materials such as contrast agent, tissue, bone, and so forth.

In one example, the volumetric angiographic image 120 that is received in the operation S110 is generated subsequent to the injection of a contrast agent into the vasculature of a patient. In this example, the volumetric angiographic image 120 represents a distribution of a contrast agent in the vascular region. The contrast agent serves to improve the contrast between blood and surrounding tissue in the volumetric angiographic image 120. In this example, the volumetric angiographic image 120 may be referred-to as a contrast-enhanced volumetric angiographic image. Contrast-enhanced volumetric angiographic images may be generated by a CT imaging system, or by an MRI system. MRI volumetric angiographic images may alternatively be generated in the absence of a contrast agent, and are often referred-to as non-contrast-enhanced MR angiography "NC-MRA" images.

With continued reference to the operation S110, the volumetric angiographic image data may be received from various sources in the operation S110. These include a medical imaging system such as one of the imaging systems described above, and also other sources such as a computer readable storage medium, the Internet, the Cloud, and so forth. In general, the volumetric angiographic image data may be received by the one or more processors 110 via any form of data communication, including via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

Referring now to the operation S120 mentioned above with reference to Fig. 1 and Fig. 2; in this operation, projection angiographic image data is received. The projection angiographic image data comprises one or more projection images $130_{1..i}$ of the vascular region.

The projection image(s) $130_{1..i}$ that are received in the operation S120 may be generated by various types of projection X-ray imaging systems. The projection X-ray imaging system may be a spectral projection X-ray imaging system. Spectral projection X-ray imaging systems generate X-ray attenuation data representing X-ray attenuation within multiple different energy intervals. The X-ray attenuation data generated by a spectral projection X-ray imaging system may be processed, e.g. using various material decomposition algorithms, in order to distinguish between media that have similar X-ray attenuation values when measured within a single energy interval, and which would be indistinguishable in X-ray attenuation data obtained using a conventional projection X-ray imaging system. Thus, X-ray attenuation data generated by a spectral projection X-ray imaging system, may be processed to provide projection images with improved specificity to materials such as contrast agent, tissue, bone, and so forth.

With continued reference to the operation S120, in one example, the projection image(s) $130_{1..i}$ that are received in the operation S120 are generated subsequent to the injection of a contrast agent into the vasculature of the patient. In this example, the projection image(s) $130_{1..i}$ represent a distribution of a contrast agent in the vascular region. The contrast agent serves to improve the contrast between blood and surrounding tissue in projection image(s) $130_{1..i}$. In this example, the projection image(s) $130_{1..i}$ may be referred-to as contrast-enhanced projection images.

The projection image(s) $130_{1..i}$ and the volumetric image 120 may relate to different stages in an interventional procedure. For instance, in one example, the projection image(s) $130_{1..i}$ of the vascular region that are received in the operation S120 are intra-procedural projection image(s) of the vascular region, and the corresponding volumetric image 120 of the vascular region is a pre-procedural volumetric image of the vascular region, or a peri-procedural volumetric image of the vascular region.

The projection image(s) $130_{1..i}$ may form a temporal series of images. For instance, in one example, a plurality of projection images $130_{1..i}$ of the vascular region are received in the operation S120 and the projection images $130_{1..i}$ form a temporal series of images representing the vascular region at different points in time.

With continued reference to the operation S120, the projection image(s) $130_{1..i}$ may be received from various sources in the operation S120. These include a medical imaging system such as one of the imaging systems described above, and also other sources such as a computer readable storage medium, the Internet, the Cloud, and so forth. In general, the volumetric angiographic image data may be received by the one or more processors 110 via any form of data communication.

It is noted that the projection image(s) $130_{1..i}$ and the volumetric image 120 described above represent the same vascular region. In some cases, fewer vessels of the vascular region may be represented in the projection image(s) $130_{1..i}$ than in the volumetric image 120. In general, some, or all, of the vessels of the vascular region represented in the volumetric image 120 may be represented in the projection image(s) $130_{1..i}$. Contrast agent that is injected into the vasculature in order to obtain the projection image(s) $130_{1..i}$ may be injected intra-arterially, for example it may be injected into a cardiac chamber, or alternatively it may be injected intra-venously. In the former case, arteries that are distal to the point of injection may be imaged. In the latter case, the contrast agent typically flows through the full venous and arterial system, and consequently all vessels may be imaged.

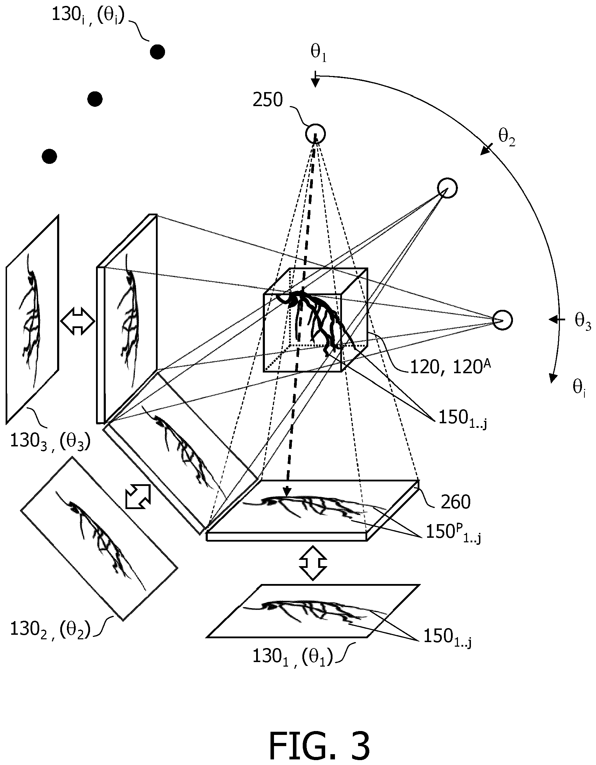

Referring now to the operation S130 mentioned above with reference to Fig. 1 and Fig. 2; in this operation, the volumetric image 120 is adapted using the one or more projection images $130_{1..i}$ to provide an adapted volumetric image $120^A$. Two implementations of the adapting operation S130 are described in more detail below. A first implementation is described with reference to Fig. 3 - Fig. 6. In this implementation, the vascular region comprises one or more vessels $150_{1..j}$, and the adapting S130 comprises adapting the one or more vessels in the volumetric image 120 to optimize a correspondence between the one or more vessels $150_{1..j}$ in each of the one or more projection images $130_{1..i}$ and a corresponding projection of the one or more vessels $150^P_{1..j}$ in the volumetric image. In a second implementation, the projection angiographic image data comprises a plurality of projection images of the vascular region; and the projection images $130_{1..i}$ represent different perspectives $\theta_{1..i}$ of the vascular region. In this implementation, the operation of adapting S130 the volumetric image 120 using the one or more projection images $130_{1..i}$, comprises: modelling the vascular region using the projection images $130_{1..i}$ to provide a model of the vascular region; and combining the model of the vascular region with the volumetric image 120 to provide the adapted volumetric image $120^A$.

Referring now to the operation S140 mentioned above with reference to Fig. 1 and Fig. 2; in this operation an image representation 140 of the vascular region, is outputted. The image representation is generated using the adapted volumetric image $120^A$. Examples of the image representation 140 include one or more of:

i) a three-dimensional rendering of the adapted volumetric image $120^A$;
ii) a projection of the adapted volumetric image $120^A$;
iii) a side by side positioning of the adapted volumetric image $120^A$, and at least one of the one or more projection images $130_{1..i}$;
iv) an overlay image comprising the adapted volumetric image $120^A$, and at least one of the one or more projection images $130_{1..i}$;
v) a schematic image representing the adapted volumetric image $120^A$.

Alternative image representations of the vascular region may likewise be generated using the adapted volumetric image $120^A$, and outputted.

The image representation 140 may be outputted in various ways in the operation S140. For instance, the image representation 140 may be outputted to a display device, such as to the display 280 illustrated in Fig. 1, or to a virtual/augmented reality display device, or to printer, or to a computer-readable storage medium, and so forth.

Thus, the system 100 outputs an image representation of the vascular region. The image representation is generated using an adapted volumetric image that is provided by adapting a volumetric image of the vascular region using the one or more projection images of the vascular region. The image representation therefore benefits from features that are present in the projection image(s) as well as features that are present in the volumetric image. In so doing, the system provides an improved visualization of the vascular region.

The system 100 may also include one or more additional features, as described in the examples below.

In one example, the one or more processors 110 are configured to register the one or more projection images $130_{1..i}$ to the volumetric image 120 prior to adapting S130 the volumetric image 120.

The registration that is performed in this operation may be a rigid registration. A two-stage registration may be used in this operation. For example, if the image acquisition parameters of the medical imaging system that generated the projection image(s) $130_{1..i}$ are known, an initial registration may be performed by using the acquisition parameters to bring the projection image(s) $130_{1..i}$ into alignment with the volumetric image 120. A subsequent registration may then be performed based on one or more landmarks that are common to the projection image(s) $130_{1..i}$ and the volumetric image 120. For example, if the vascular region represents the heart, landmarks such as the trunk of a vascular tree, or a bifurcation such as the bifurcation defining the distal end of the left coronary artery, may be used. The subsequent registration may be performed by using an algorithm to match features in the projection image(s) $130_{1..i}$ to corresponding features in projections of the volumetric image 120, and then registering the images using the matching features. The subsequent registration may be a semantic registration wherein vessel segments are identified (e.g. Left-Main, LAD, LCX, RCA, etc.) in both the volumetric image 120 and in the projection image(s) $130_{1..i}$. A semantic registration makes use of this dual identification to bring into correspondence vessels that share the same identification (label) in the volumetric image 120 and in the projection image(s) $130_{1..i}$. Semantic registration is typically achieved with a U-net, or another type of Neural Networks. Alternatively, the subsequent registration may be performed by using a neural network to predict a perspective of the vascular region represented by the projection image(s) $130_{1..i}$, and then using the predicted perspective to register the images.

Referring now to the first implementation of the adapting operation S130 mentioned above with reference to Fig. 1 and Fig. 2; in this implementation, the vascular region comprises one or more vessels $150_{1..j}$, and the adapting S130 comprises adapting the one or more vessels in the volumetric image 120 to optimize a correspondence between the one or more vessels $150_{1..j}$ in each of the one or more projection images $130_{1..i}$ and a corresponding projection of the one or more vessels $150^P_{1..j}$ in the volumetric image.

This example is illustrated schematically in Fig. 3, which is a schematic diagram illustrating an example of the evaluation of a correspondence between the vessels $150_{1..j}$ in a plurality of projection images $130_{1..i}$ and a corresponding projection of the vessels $150^P_{1..j}$ in a volumetric image 120, in accordance with some aspects of the present disclosure. A volumetric image 120 of a vascular region is illustrated in the center of Fig. 3. The volumetric image 120 includes vessels $150_{1..i}$. In this example, the vascular region is the left branch of the coronary tree, and includes vessels such as the left coronary artery. The volumetric image 120 may be generated by a volumetric imaging system such as a CT imaging system, an MRI system, or a 3D ultrasound imaging system, for example. Multiple projection images $130_{1..i}$ are also illustrated in Fig. 3. The projection images $130_{1..i}$ represent corresponding perspectives $\theta_{1..i}$ of the vascular region. The perspectives $\theta_{1..i}$ are defined by the orientation of an X-ray source 250 and an X-ray detector 260 of a projection X-ray imaging system 270 (illustrated in Fig. 1) with respect to the vascular region. The projection images $130_{1..i}$ may be obtained for their corresponding perspectives $\theta_{1..i}$ by positioning, e.g. by rotating, the X-ray source 250 and X-ray detector 260 of the projection X-ray imaging system 270 (illustrated in Fig. 1) with respect to the vascular region so as to provide the orientations defined by the angles $\theta_{1..i}$. For each of the projection images $130_{1..i}$, Fig. 3 also illustrates the corresponding projection of the one or more vessels $150^P_{1..j}$ in the volumetric image. For instance, for the projection image $130_1$, and which represents a perspective of the vascular region defined by $\theta_1$, the corresponding projection of the one or more vessels $150^P_{1..j}$ in the volumetric image is illustrated immediately above the projection image $130_1$. The projections of the one or more vessels $150^P_{1..j}$ in the volumetric image may be obtained by projecting the volumetric image 120 onto a virtual X-ray detector with virtual X-rays emitted from a virtual X-ray source wherein the virtual X-ray source and the virtual X-ray detector are arranged at the same perspective with respect to the vascular region as that at which the projection image was obtained. The orientations $\theta_{1..i}$ may be known, or determined, as described in the registration operation described above.

The evaluation of the correspondence between the one or more vessels $150_{1..j}$ in the projection image(s) $130_{1..i}$,

and the corresponding projection of the one or more vessels $150^P_{1..j}$ in the volumetric image 120, is illustrated in Fig. 3 by way of the double-headed arrows. The correspondence may be evaluated for a given pair of images using known image comparison metrics. For instance, shape, contour and "vesselness" indices can be used in this correspondence evaluation. Such indices can be applied on vessel contours, or on vessel centerlines. Likewise, vessel diameters can be compared, or a radiometric comparison may be used where the vessel contrast is locally extracted in the projection images (e.g. using differential, or morphological operations or neural network estimations). The contrast of the projected volumetric vessels can typically be estimated by Digital Reconstruction Radiography techniques "DRR".

The one or more vessels in the volumetric image 120 are then adapted in order to optimize the correspondence between the one or more vessels $150_{1..j}$ in each of the one or more projection images $130_{1..i}$ and the corresponding projection of the one or more vessels $150^P_{1..j}$ in the volumetric image. This optimization may be performed globally for multiple projection image(s) $130_{1..i}$ so as to provide a global optimum correspondence between the projection image(s) $130_{1..i}$ and their corresponding projections of the volumetric image 120. The global optimum may be determined by performing an optimization based on a value of a cost function that represents a combination of the individual image comparison metrics representing the correspondence(s) between the one or more vessels $150_{1..j}$ in the projection image(s) $130_{1..i}$, and the corresponding projection of the one or more vessels $150^P_{1..j}$ in the volumetric image 120.

By adapting the one or more vessels in the volumetric image 120 to optimize a correspondence between the one or more vessels $150_{1..j}$ in each of the one or more projection images $130_{1..i}$ and a corresponding projection of the one or more vessels $150^P_{1..j}$ in the volumetric image, this example provides an adapted volumetric image $120^A$ that benefits from information in the projection image(s) $130_{1..i}$. For instance, the spatial resolution of the volumetric image 120 is often relatively lower than the spatial resolution of the projection image(s) $130_{1..i}$. Consequently, optimizing the correspondence enables the transfer of information that is poorly represented, or not represented at all in the volumetric image 120, into the adapted volumetric image $120^A$ and hence into the image representation that is generated using the adapted volumetric image $120^A$.

In one example, the projection angiographic image data includes a plurality of projection images $130_{1..i}$ of the vascular region; and the projection images $130_{1..i}$ represent different perspectives $\theta_{1..i}$ of the vascular region. In this case, optimizing the correspondence, facilitates the adapted volumetric image $120^A$ to benefit from additional details that are provided by the different perspectives of the vascular region.

In another example, the volumetric image 120 is generated at an earlier point in time to the projection images $130_{1..i}$. For instance, the volumetric image 120 of the vascular region may be a pre-procedural volumetric image of the vascular region, or a peri-procedural volumetric image of the vascular region; and the one or more projection images of the vascular region may be intra-procedural projection images of the vascular region. In this case, optimizing the correspondence, facilitates the adapted volumetric image $120^A$ to benefit from changes in the vasculature, such as deformations in the vasculature, vessel movement as result of patient re-positioning, and so forth that may have occurred since the time of the volumetric image 120. In this example the adapted volumetric image $120^A$ is therefore an updated version of the volumetric image 120. Providing an adapted volumetric image $120^A$, and hence an image representation, in this manner has a significant advantage over current practice because it may be impossible to transfer a patient to a volumetric imaging system to obtain an updated volumetric image during an interventional procedure.

Various examples of the operation of adapting S130 the volumetric image 120 are described in detail below. In general, the adapting operation may include adaptations such as deforming a path of the one or more vessels $150_{1..j}$ in the volumetric image, or extending the one or more vessels $150_{1..j}$ in the volumetric image 120, or deforming a cross sectional shape 160 of the one or more vessels $150_{1..j}$ in the volumetric image 120, or deforming a cross sectional shape 160 and/or adjusting an intensity 190 within the cross sectional shape 160 of the one or more vessels $150_{1..j}$ in the volumetric image 120. For instance, in one example, the adapting S130 comprises:

i) deforming a path of the one or more vessels $150_{1..j}$ in the volumetric image 120 to optimize a correspondence between a path of the one or more vessels $150_{1..j}$ in each of the one or more projection images $130_{1..i}$ and a corresponding projection of the path of the one or more vessels $150^P_{1..j}$ in the volumetric image.

In this example, the adapted volumetric image $120^A$ takes account of deformations in the projection images. Over time, vessels in the projection images may deform as a result of cardiac motion, breathing motion, patient re-positioning, or the presence of an interventional device in a vessel. By taking account of such deformations in the adapted volumetric image $120^A$, the image representation 140, which is generated using the adapted volumetric image $120^A$, is updated to provide a more accurate view of the vascular region. Updating the image representation in this manner also helps a physician to visualize the correspondence between the projection angiographic images and the volumetric angiographic image. Elastic image registration techniques may be used in this example to deform the path of the one or more vessels $150_{1..j}$ in the volumetric image. For example, the path may be deformed iteratively based on the value of the cost function described above until a stopping criterion is met. The stopping criterion may for instance represent that a minimum value of the cost function has been obtained, or that the value of the cost function is below a threshold value, or that no further improvement of the value of the cost function has been obtained over a specified number of iterations.

In another example, the adapting S130 comprises:

ii) extending the one or more vessels $150_{1..j}$ in the volumetric image 120 to optimize a correspondence between the one or more vessels $150_{1..j}$ in each of the one or more projection images $130_{1..i}$ and a corresponding projection of the one or more vessels $150^P_{1..j}$ in the volumetric image.

As mentioned above, the spatial resolution of volumetric images is often relatively lower than the spatial resolution of projection images. Consequently, narrow vessels that are visible in projection images may be poorly visible, or invisible in volumetric images. In this example, one or more vessels $150_{1..j}$ in the volumetric image 120 are extended. By adapting the volumetric image in this manner, the adapted volumetric image $120^A$, and consequently the image representation, extends the paths of vessels, and consequently improves the correspondence between the projection angiographic images and the volumetric angiographic image. In-turn, the improved image representation provides improved guidance to a physician.

In another example, the adapting S130 comprises:

iii) deforming a cross sectional shape 160 of the one or more vessels $150_{1..j}$ in the volumetric image 120 to optimize a correspondence between a dimension $170_i$ of a cross section through the one or more vessels $150_{1..j}$ in each of the one or more projection images $130_{1..i}$ and a dimension $170^P_i$ of a corresponding projection of the cross sectional shape of the one or more vessels in the volumetric image.

As mentioned above, the spatial resolution of volumetric images is often relatively lower than the spatial resolution of projection images. Consequently, the cross sectional shape of vessels in volumetric images may be inaccurate. As a result, the cross sectional dimensions of vessels such as the vessel diameter, vessel cross sectional area, vessel lumen area, and so forth, may also be inaccurate. Such inaccuracies hamper the reliability of image-based assessments of blood flow in the vessel, the reliability of stent size estimations, and so forth. The cross sectional shapes of vessels are not determinable from projection images. However, the dimensions of cross sections through vessels can be determined reliably in projection images. In this example, by deforming the cross sectional shape of the vessel(s) in the volumetric image so as to optimize a correspondence between a dimension $170_i$ of a cross section through the one or more vessels $150_{1..j}$ in each of the one or more projection images $130_{1..i}$ and a dimension $170^P_i$ of a corresponding projection of the cross sectional shape of the one or more vessels in the volumetric image, a more accurate representation of the cross sectional shape of the vessel(s) is obtained in the adapted volumetric image $120^A$. Thus, this example provides a more accurate adapted volumetric image $120^A$, and consequently a more accurate image representation, which in-turn provides improved guidance to a physician.

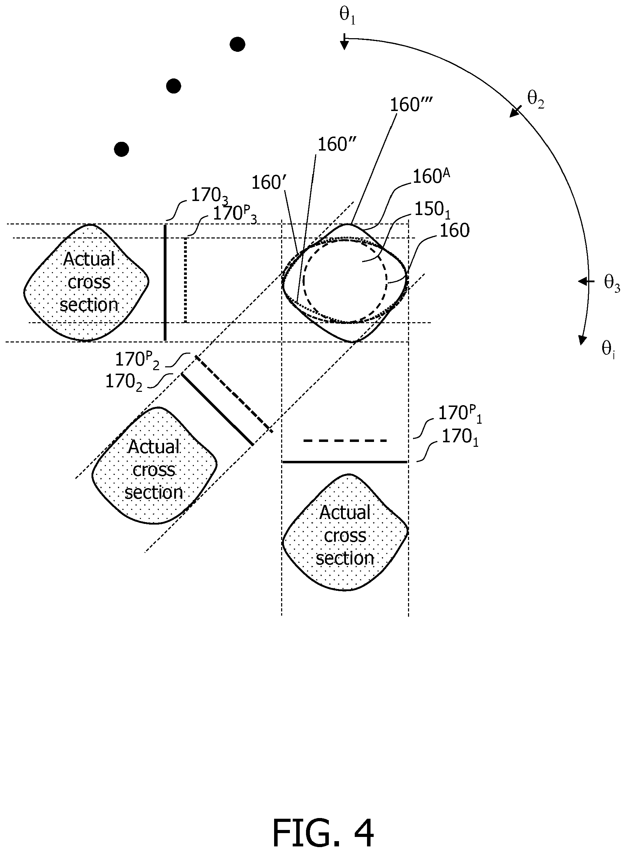

This example is described with reference to Fig. 4, which is a schematic diagram illustrating a first example of the deforming of a cross sectional shape 160 of a vessel $150_1$ in a volumetric image, in accordance with some aspects of the present disclosure. In the center of Fig. 4, a vessel $150_1$ from within the volumetric image 120 in Fig. 3, is illustrated as having a circular cross sectional shape 160. The circular cross sectional shape 160 is illustrated via long-dashed lines. For simplicity, the center of the vessel $150_1$ corresponds to the isocenter of the projection X-ray imaging system defined by the X-ray source 250 and the X-ray detector 260, in Fig. 3. Thus, the perspectives $\theta_{1..i}$ in Fig. 4 correspond to the same perspectives illustrated in Fig. 3. For each of the perspectives $\theta_{1..i}$ in Fig. 4, there is shown opposite the labelled perspective, a corresponding projection of the cross sectional shape of the vessel $150_1$ in the volumetric image. The projections are shown by way of dashed thick black lines, and the projections have corresponding dimensions $170^P_{1..i}$. The projections of the vessels illustrated in Fig. 4 may be obtained in the same manner as described above with reference to Fig. 3, i.e. by projecting the cross section 160 of the vessel $150_1$ in the volumetric image 120 onto a virtual X-ray detector with virtual X-rays emitted from a virtual X-ray source wherein the virtual X-ray source and the virtual X-ray detector are arranged at the relevant perspective $\theta_{1..i}$ with respect to the vascular region. For instance, for the perspective $\theta_1$, the projection shown by way of dashed thick black lines in the lower portion of Fig. 4 is obtained by projecting the long-dashed circular cross section 160 of the vessel $150_1$ in the volumetric image 120 onto a virtual X-ray detector with virtual X-rays emitted from a virtual X-ray source wherein the virtual X-ray source and the virtual X-ray detector are arranged at the perspective $\theta_1$ with respect to the vascular region.

In the illustrated example, the dimensions $170_{1..i}$ represent a vessel width. The dimensions $170^P_{1..i}$ of the projections of the vessels illustrated in Fig. 4 correspond to the dimensions of the projections of the one or more vessels $150^P_{1..j}$ in the volumetric image illustrated in Fig. 3. For the purposes of illustration, the vessel $150_1$ in the center of Fig. 4 is enlarged as compared to the vessels in the center of Fig. 3. Consequently, the dimensions $170_{1..i}$ of the vessel illustrated in Fig. 4 are also enlarged as compared to the dimensions of the projected vessels in the $150^P_{1..j}$ illustrated in Fig. 3.

In the lowermost portion of Fig. 4, the Actual cross section of the vessel $150^P_{1..j}$, is shown. This is the real profile, which it is desired to replicate by deforming the cross sectional shape 160 of the vessel $150_1$ in the volumetric image 120. For the perspective $\theta_1$, the cross sectional shape 160 of the vessel $150_1$ in the center of Fig. 4 is then deformed, in this example, perpendicularly with respect to the perspective $\theta_1$, i.e. horizontally with respect to the drawing, such that the dimension $170_1$ of the cross section through the vessel $150_1$ in the projection image $130_1$ corresponds to the dimension $170^P_1$ of the projection of the cross sectional shape of the vessel $150_1$ in the volumetric image. For the perspective $\theta_1$, this

condition is met when the cross sectional shape of the deformed vessel $150_1$, i.e. 160', has a width in the horizontal direction that matches the width of the Actual cross section between the indicated dashed lines. This condition may be met by elastically deforming the cross sectional shape of the vessel 160. For the perspective $\theta_1$, this deformation results in the cross sectional shape 160' through the vessel $150_1$, i.e. an ellipse with the long axis in the horizontal direction. This deformation provides a cross sectional shape 160' of the vessel $150_1$ in the center of Fig. 4 that is closer to the Actual cross section. The same operations are performed for each of the perspectives $\theta_{2..i}$, in each case deforming the cross sectional shape 160 of the vessel $150_1$ such that the dimension $170_{2..i}$ of the cross section through the vessel $150_1$ in the projection image $130_{2..i}$ corresponds to the dimension $170^P_{2..i}$ of the projection of the cross sectional shape of the vessel $150_1$ in the volumetric image. For the perspective $\theta_2$, this results in the cross sectional shape 160" with a cross sectional dimension $170_2$, and for the perspective $\theta_3$, this results in the cross sectional shape 160''' with a cross sectional dimension $170_3$. Performing this operation for the perspectives $\theta_{1..i}$ results in the optimized cross sectional shape $160^A$ for the vessel $150_1$.

Whilst the deformations were described above as being performed sequentially for the perspectives $\theta_{1..i}$, it is noted that the optimization that is performed in this example, i.e. the optimizing a correspondence between a dimension $170_i$ of a cross section through the one or more vessels $150_{1..j}$ in each of the one or more projection images $130_{1..i}$ and a dimension $170^P_i$ of a corresponding projection of the cross sectional shape of the one or more vessels in the volumetric image, may be performed in a different manner. For example, the deformations may be performed simultaneously for different perspectives rather than sequentially. The effect of the optimization is to globally maximize the correspondence between a dimension $170_i$ of a cross section through the one or more vessels $150_{1..j}$ in each of the one or more projection images $130_{1..i}$ and a dimension $170^P_i$ of a corresponding projection of the cross sectional shape of the one or more vessels in the volumetric image.

In another example, the adapting S130 comprises:

iv) deforming a cross sectional shape 160 and/or adjusting an intensity 190 within the cross sectional shape 160 of the one or more vessels $150_{1..j}$ in the volumetric image 120 to optimize a correspondence between an intensity profile $180_1$ of a cross section through the one or more vessels $150_{1..j}$ in each of the one or more projection images $130_{1..i}$ and an intensity profile $180^P_1$ of a corresponding projection of the intensity within the cross sectional shape of the one or more vessels in the volumetric image.

As mentioned above, the spatial resolution of volumetric images is often relatively lower than the spatial resolution of projection images. Consequently, the cross sectional shape of vessels in volumetric images may be inaccurate. Such inaccuracies hamper the reliability of image-based assessments of blood flow in the vessel, and the reliability of stent size estimation, and so forth. As mentioned above, the cross sectional shapes of vessels are not determinable from projection images. However, the intensity profiles of cross sections through vessels can be determined reliably in projection images. In this example, by deforming a cross sectional shape 160 and/or adjusting an intensity 190 within the cross sectional shape 160 of the one or more vessels $150_{1..j}$ in the volumetric image 120 to optimize a correspondence between an intensity profile $180_1$ of a cross section through the one or more vessels $150_{1..j}$ in each of the one or more projection images $130_{1..i}$ and an intensity profile $180^P_1$ of a corresponding projection of the intensity within the cross sectional shape of the one or more vessels in the volumetric image, a more accurate representation of the cross sectional shape of the vessel(s) is obtained in the adapted volumetric image $120^A$. Similarly, a more accurate intensity within the vessel(s), i.e. a more accurate contrast agent distribution within the vessel(s), is obtained in the adapted volumetric image $120^A$. Thus, this example provides a more accurate adapted volumetric image $120^A$, and consequently a more accurate image representation 140, which in-turn provides improved guidance to a physician.

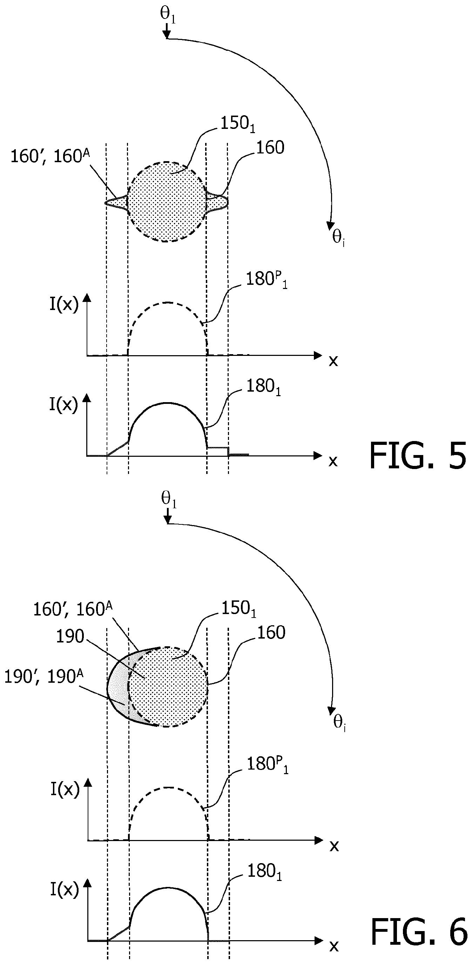

This example is described with reference to Fig. 5, and Fig. 6. Fig. 5 is a schematic diagram illustrating a second example of the deforming of a cross sectional shape 160 of a vessel $150_1$ in a volumetric image, in accordance with some aspects of the present disclosure. In the center of Fig. 5, a vessel $150_1$ from within the volumetric image 120 in Fig. 3, is illustrated as having a circular cross sectional shape 160. The circular cross sectional shape 160 is illustrated via long-dashed lines. For simplicity, the center of the vessel $150_1$ in Fig. 5 corresponds to the isocenter of the projection X-ray imaging system defined by the X-ray source 250 and the X-ray detector 260, in Fig. 3. Thus, the perspective $\theta_1$ in Fig. 5 corresponds to the same perspective $\theta_1$ illustrated in Fig. 3. Only one perspective, $\theta_1$, is illustrated in Fig. 5 for simplicity. For the perspectives $\theta_1$ in Fig. 5, there is shown opposite the labelled perspective, an intensity profile $180^P_1$ of a corresponding projection of the intensity within the cross sectional shape of the vessel $150_1$. The intensity profile $180^P_1$ is shown by way of a dashed thick black line. The intensity profile $180^P_1$ may be obtained in a similar manner as described above with reference to Fig. 3; in this case by integrating the intensity within the cross sectional shape of the vessel $150_1$ in the volumetric image 120, along the paths of virtual X-rays emitted from a virtual X-ray source to provide an integrated intensity on a virtual X-ray detector, the virtual X-ray source and the virtual X-ray detector being arranged at the relevant perspective $\theta_1$ with respect to the vascular region. Although only one perspective, $\theta_1$, is illustrated in Fig. 5, the operations described above may be performed in a similar manner for the further perspectives $\theta_{2..i}$. In the example illustrated in Fig. 5, for a uniform intensity distribution with density $\delta$ within a vessel $150_1$ having a circular cross section with radius r, and ignoring X-ray beam divergence for simplicity, the intensity profile is given by:

$$p(x) = 2 \cdot \delta \cdot \sqrt{r^2 - x^2}$$

Equation 1

In the lowermost portion of Fig. 5, the intensity profile $180_1$ of a cross section through the vessel $150_1$ in the projection image $130_1$ that is obtained from the perspective $\theta_1$, is shown. For the purposes of illustration, an example is shown in which the intensity profile $180_1$ is asymmetric. The intensity profile $180_1$ is the real profile, which it is desired to replicate by deforming the cross sectional shape 160 of the vessel $150_1$ in the volumetric image 120. For the perspective $\theta_1$, the cross sectional shape 160 of the vessel $150_1$ in the center of Fig. 5 is then deformed, in this case whilst maintaining a uniform intensity distribution with density $\delta$ within the vessel $150_1$, such that the intensity profile $180_1$ of the cross section through the vessel $150_1$ in the projection image $130_1$ corresponds to the intensity profile $180^P_1$ of the corresponding projection of the intensity within the cross sectional shape of the vessel $150_1$. For the perspective $\theta_1$, this condition is met when the cross sectional shape of the deformed vessel $150_1$, has the shape 160' illustrated in Fig. 5. This condition may be met by elastically deforming the cross sectional shape of the vessel 160. In the example illustrated in Fig. 5, only a single perspective $\theta_1$, is illustrated. However, corresponding deformations may also be performed for the further perspectives $\theta_{2..i}$ in order to globally maximize the correspondence between the intensity profile $180_1$ of a cross section through the one or more vessels $150_{1..j}$ in each of the one or more projection images $130_{1..i}$ and an intensity profile $180^P_1$ of a corresponding projection of the intensity within the cross sectional shape of the one or more vessels in the volumetric image. The result of this global optimization is that the vessel $150_1$, has the cross sectional shape $160^A$.

In the example described with reference to Fig. 5, the cross sectional shape of the vessel(s) was deformed whilst maintaining a uniform intensity distribution with density $\delta$ within the vessel $150_1$. In another example, instead of, or in addition to, deforming the cross sectional shape 160 of the vessel(s) $150_{1..j}$, the intensity 190 may be adjusted within the cross sectional shape 160 of the one or more vessels. This example is described with reference to Fig. 6, which is a schematic diagram illustrating an example of the adjusting of an intensity 190 within a cross sectional shape 160 of a vessel $150_1$ in a volumetric image, in accordance with some aspects of the present disclosure.

In the center of Fig. 6, a vessel $150_1$ from within the volumetric image 120 in Fig. 3, is illustrated as having a circular cross sectional shape 160. The circular cross sectional shape 160 is illustrated via long-dashed lines. For simplicity, the center of the vessel $150_1$ in Fig. 6 corresponds to the isocenter of the projection X-ray imaging system defined by the X-ray source 250 and the X-ray detector 260, in Fig. 3. Thus, the perspectives $\theta_1$ in Fig. 6 corresponds to the same perspective $\theta_1$ illustrated in Fig. 3. Only one perspective, $\theta_1$, is illustrated in Fig. 6 for simplicity. For the perspectives $\theta_1$ in Fig. 6, there is shown opposite the labelled perspective, an intensity profile $180^P_1$ of a corresponding projection of the intensity within the cross sectional shape of the vessel $150_1$. The intensity profile $180^P_1$ is shown by way of a dashed thick black line. The intensity profile $180^P_1$ may be obtained in a similar manner as described above with reference to Fig. 3, in this case by integrating the intensity within the cross sectional shape of the vessel $150_1$ in the volumetric image 120, along the paths of virtual X-rays emitted from a virtual X-ray source to provide an integrated intensity on a virtual X-ray detector, the virtual X-ray source and the virtual X-ray detector being arranged at the relevant perspective $\theta_1$ with respect to the vascular region. Although only one perspective, $\theta_1$, is illustrated in Fig. 6, the operations described above may be performed in a similar manner for the further perspectives $\theta_{2..i}$. In the example illustrated in Fig. 6, a uniform intensity distribution 190 with density $\delta$ is assumed within the vessel $150_1$, and the vessel has a circular cross section.

In the lowermost portion of Fig. 6, the intensity profile $180_1$ of a cross section through the vessel $150_1$ in the projection image $130_1$ that is obtained from the perspective $\theta_1$, is shown. The intensity profile $180_1$ is the real profile, which it is desired to replicate by both deforming the cross sectional shape 160 of the vessel $150_1$ and changing the intensity within the cross sectional shape of the vessel $150_1$. For the perspective $\theta_1$, the cross sectional shape 160 of the vessel $150_1$ in the center of Fig. 6 is then deformed, and also the intensity distribution within the vessel $150_1$ is also adjusted, such that the intensity profile $180_1$ of the cross section through the vessel $150_1$ in the projection image $130_1$ corresponds to the intensity profile $180^P_1$ of the corresponding projection of the intensity within the cross sectional shape of the vessel $150_1$. For the perspective $\theta_1$, this condition is met when the cross sectional shape of the deformed vessel $150_1$, has the shape 160' illustrated in Fig. 6 and the intensity distribution 190' is provided. This condition may be met by elastically deforming the cross sectional shape of the vessel 160, and also adjusting the intensity within the adjusted cross sectional shape 160' of the vessel $150_1$. In the example illustrated in Fig. 6, only a single perspective $\theta_1$, is illustrated. However, corresponding deformations, and corresponding adjustments to the intensity distribution, may also be performed for the further perspectives $\theta_{2..i}$ in order to globally maximize the correspondence between the intensity profile $180_1$ of a cross section through the one or more vessels $150_{1..j}$ in each of the one or more projection images $130_{1..i}$ and an intensity profile $180^P_1$ of a corresponding projection of the intensity within the cross sectional shape of the one or more vessels in the volumetric image. The result of this global optimization is that the vessel $150_1$, has the cross sectional shape $160^A$ and the intensity distribution $190^A$.

Whilst the operations described above were described for a single vessel $150_1$, and in some cases for a single cross section through the vessel $150_1$, it is to be appreciated that these operations may also be performed for further vessels $150_{2..j}$, and also for further cross sections along the vessel(s) in a similar manner.

In one example, the operations described above are performed for a temporal series of images that represent the vascular region at different points in time. In this example, the projection angiographic image data comprises a plurality of projection images, and the projection images $130_{1..i}$ form a temporal series of images representing the vascular region at different points in time. The one or more processors 110 are configured to perform the adapting S130, and the outputting S140, for each image in the temporal series such that the outputted image representation 140 is updated throughout the temporal series of images.

Thus, this example updates the image representation 140 over time. In so doing, changes to the vascular region over time, such as for example vessel movement and deformation due to the presence of an interventional device in the vessel, patient re-positioning, and so forth, are accounted for in the image representation 140. This continual improvement of the image representation 140 provides improved guidance to the physician.

In a related example, the operation of outputting S140 an image representation 140 comprises simultaneously outputting the image representation generated at each of a plurality of the points in time for comparing the image representations generated at the different points in time.

This example facilitates an evaluation of temporal changes in the vascular region. By way of an example, the image representation may be outputted at difference stages in an interventional procedure, such as pre- and post-stenting, or pre- and post-balloon inflation. By providing the physician with the ability to evaluate such changes, the physician may assess the progress of the interventional procedure.

In another related example, the temporal series of images represent the vascular region in one or more cardiac phases, and the adapting S130, and the outputting S140, are performed selectively for the images corresponding to each of one or more cardiac phases.

By performing these operations selectively for the images corresponding to each of one or more cardiac phases, this example reduces the amount of cardiac motion, and also cardiac motion-induced vessel deformation, in the vascular region between the projection images. This, in-turn results in a more accurate image representation 140 of the vascular region.

As mentioned above, a second implementation of the adapting operation S130 illustrated in Fig. 2, is also contemplated. In this implementation, the projection angiographic image data comprises a plurality of projection images of the vascular region; and the projection images $130_{1..i}$ represent different perspectives $\theta_{1..i}$ of the vascular region. In this implementation, the operation of adapting S130 the volumetric image 120 using the one or more projection images $130_{1..i}$, comprises:

modelling the vascular region using the projection images $130_{1..i}$ to provide a model of the vascular region; and combining the model of the vascular region with the volumetric image 120 to provide the adapted volumetric image $120^A$.

In this implementation, the operation of modelling the vascular region may be performed using techniques disclosed in a document by Cimen, S., et al., "Reconstruction of coronary arteries from X-ray angiography: A review", Med Image Anal. 2016, Aug:32:46-68, doi: 10.1016/j.media.2016.02.007. This document describes methods of computing reconstructions of coronary arteries from X-ray angiography. The term "model" in this context encompasses generating a volumetric representation of the vascular region from a range of different numbers of different perspectives. The number of views extends from a so-called sparse reconstruction that is made using a limited number of different perspectives, i.e. two, or more, perspectives, to a so-called tomographic reconstruction which is made using a large number of different perspectives. Clearly, the higher the number of perspectives, the greater the amount of information that may be incorporated into the adapted volumetric image $120^A$, and hence the greater the accuracy of the resulting image representation 140. A tomographic reconstruction of a volumetric image that is obtained using a projection X-ray imaging system is sometimes referred-to as a 3D rotational angiogram "3DRA".

In this implementation, the operation of combining the model of the vascular region with the volumetric image 120, may include operations such as overlaying the model with the volumetric image, and fusing the model with the volumetric image.

In one example, the vascular region comprises one or more vessels $150_{1..j}$, and the operation of combining the model of the vascular region with the volumetric image 120 comprises:

comparing the modelled vascular region with the volumetric image 120 to identify one or more one or more vessels in the modelled vascular region that are missing from the volumetric image 120 or poorly represented in the volumetric image; and
augmenting the volumetric image 120 with the one or more missing, or poorly represented, vessels to provide the adapted volumetric image $120^A$.

This results in the provision of a more extensive vascular region, and in a similar manner to the first implementation of the adapting operation S130 described above, it likewise provides improved guidance to a physician. The image representation in this second implementation may be outputted in a similar manner to that described above for the first implementation.

In either of the two implementations of the adapting operation S130 described above, one or more further operations may also be performed, as described in the examples below.

In one example, the value(s) of vessel parameters are calculated. In this example, the vascular region comprises one or more vessels $150_{1..j}$, and the one or more processors 110 are configured to:

calculate, from the adapted volumetric image $120^A$, or from the image representation 140, a value of one or more vessel parameters 210 for the one or more vessels $150_{1..j}$; and
output the calculated value of the one or more vessel parameters, or a value of one or more parameters derived therefrom.

The value(s) of the vessel parameter(s) may be determined from the adapted volumetric image $120^A$, or from the image representation 140, by measurement of the relevant parameter. This may involve segmenting the adapted volumetric image $120^A$, or the image representation 140, using known segmentation techniques.

Examples of vessel parameters that may be calculated in accordance with this example include a vessel length, diameter, or cross sectional area; a vessel lumen length, diameter, or cross sectional area; an occlusion length, diameter, or cross sectional area; a stenosis fraction, a measurement or a composition of a plaque deposit in the vessel, a morphology of a plaque deposit in the vessel, an angle between branches of a bifurcation in a vessel, and so forth. The vessel parameters may also be used to derive the values of other parameters, such as for example the value of a blood flow parameter. The values of blood flow parameters such as the blood flow rate, the fractional flow reserve "FFR" , the instantaneous wave-free ratio "iFR", the Coronary Flow Reserve "CFR", the Thrombolysis in Myocardial Infarction "TIMI" flow grade, the Index of Microvascular Resistance "IMR", and the Hyperemic Microvascular Resistance index "HMR", and so forth, may be derived. Such blood flow parameter may be evaluated using a haemodynamic model, using, as input, the values of the vessel parameters described above.

The calculated value(s) of the vessel parameter(s) may be outputted in various ways in this example. For instance, the value(s) may be outputted to a display device, such as to the display 280 illustrated in Fig. 1, or to a virtual/augmented reality display device, or to printer, or to a computer-readable storage medium, and so forth.

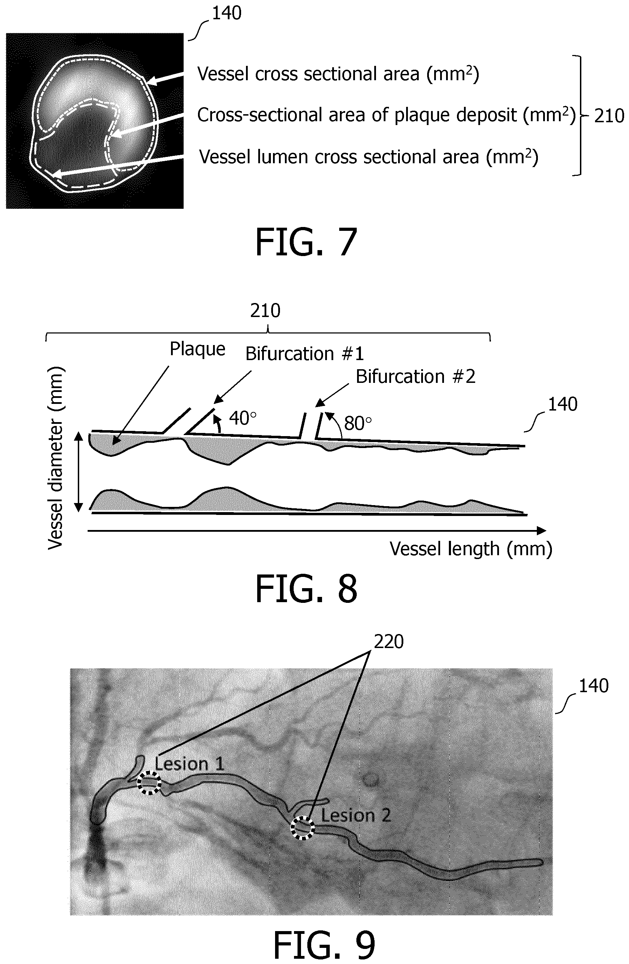

By way of an example, Fig. 7 is a schematic diagram illustrating a first example of the calculated values of some example vessel parameters 210, in accordance with some aspects of the present disclosure. In the example illustrated in Fig. 7, the vessel parameters are determined from the adapted volumetric image $120^A$ and the vessel parameters are displayed in an image representation 140 that includes a cross section through a vessel in the adapted volumetric image $120^A$. In this example, the vessel parameters include the vessel cross sectional area, the vessel lumen cross sectional area, and the cross-sectional area of a plaque deposit in the vessel.

By way of another example, Fig. 8 is a schematic diagram illustrating a second example of the calculated values of some example vessel parameters, in accordance with some aspects of the present disclosure. In the example illustrated in Fig. 8, the vessel parameters are determined from the adapted volumetric image $120^A$ and the vessel parameters are displayed in a schematic image representing the adapted volumetric image $120^A$. In this example, the vessel parameters include the vessel diameter at a plurality of positions along the vessel, a thickness of a plaque deposit in the vessel, and the angle between branches of bifurcations in the vessel.

In a related example, the operation of outputting S140 the image representation 140 includes indicating, in the image representation 140, an origin 220 of the calculated value of the one or more vessel parameters, or the calculated value of the one or more parameters derived therefrom.

The origin of the calculated value of the one or more vessel parameters may be indicated in various ways, such as using an icon, a marker, a heatmap, and so forth. Fig. 9 is an example of an image representation 140 of a vascular region including an indication of the origin of the calculated value of a vessel parameter, in accordance with some aspects of the present disclosure. In the example illustrated in Fig. 9, the origins 220 of two lesions in the vasculature are indicated by way of circular markers. Indicating the origin of such measurements may enables a physician to better plan a procedure. For instance, in the example illustrated in Fig. 9 it may enable a physician to plan suitable locations for a stent, and to subsequently navigate a balloon catheter to the planned locations.

In another example the vascular region comprises one or more vessels $150_{1..j}$, and the one or more processors 110 are configured to:

i) determine, using the adapted volumetric image $120^A$, or the image representation 140, a recommended position 230 for a stent in at least one of the one or more vessels $150_{1..j}$; and
output an indication of the recommended position for the stent;
and/or

ii) calculate, using the adapted volumetric image 120$^A$, or the image representation 140, a value of a blood flow parameter for at least one of the one or more vessels 150$_{1..j}$; and
output the value of the blood flow parameter.

In this example, the recommended position for a stent may be determined based on the vessel parameters. For instance, vessel parameters such as the vessel lumen diameter, the thickness of a plaque in the vessel, the length of a lesion, and so forth, may be used.

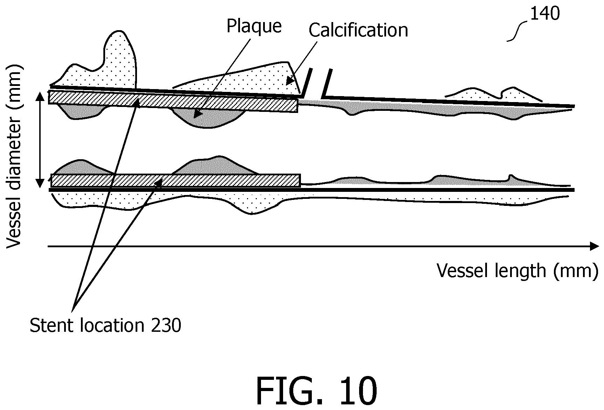

Fig. 10 is a schematic diagram illustrating an example of an image representation 140 of a vascular region including an indication of a recommended position for a stent, in accordance with some aspects of the present disclosure. Examples of blood flow parameters that may be evaluated in accordance with this example include the blood flow rate, the fractional flow reserve "FFR" , the instantaneous wave-free ratio "iFR", the Coronary Flow Reserve "CFR", the Thrombolysis in Myocardial Infarction "TIMI" flow grade, the Index of Microvascular Resistance "IMR", and the Hyperemic Microvascular Resistance index "HMR", and so forth. By way of an example, the document WO2016/001017 A1 describes the calculation of an FFR value from projection angiographic image data.

It is noted that the system 100 described above may also include one or more of: a projection imaging system for providing the projection angiographic image data, such as for example the projection X-ray imaging system 270 illustrated in Fig. 1; an injector (not illustrated in Fig. 1) for injecting a contrast agent into the vasculature of a patient; a display, such as the display 280 illustrated in Fig. 1, for displaying the image representation 140, and also other outputs generated by the one or more processors 110; a patient bed 290; and a user input device (not illustrated in Fig. 1) configured to receive user input in relation to the operations performed by the one or more processors 110, such as a keyboard, a mouse, a touchscreen, and so forth.

In another example, a computer-implemented method of providing an image representation of a vascular region, is provided. The method comprises:

receiving S110 volumetric angiographic image data comprising a volumetric image 120 of the vascular region;
receiving S120 projection angiographic image data comprising one or more projection images 130$_{1..i}$ of the vascular region;
adapting S130 the volumetric image 120 using the one or more projection images 130$_{1..i}$ to provide an adapted volumetric image 120$^A$; and
outputting S140 an image representation 140 of the vascular region, the image representation being generated using the adapted volumetric image 120$^A$.

In another example, a computer program product is provided. The computer program product comprises instructions which when executed by one or more processors 110, cause the one or more processors to carry out a method of providing an image representation of a vascular region. The method comprises:

receiving S110 volumetric angiographic image data comprising a volumetric image 120 of the vascular region;
receiving S120 projection angiographic image data comprising one or more projection images 130$_{1..i}$ of the vascular region;
adapting S130 the volumetric image 120 using the one or more projection images 130$_{1..i}$ to provide an adapted volumetric image 120$^A$; and
outputting S140 an image representation 140 of the vascular region, the image representation being generated using the adapted volumetric image 120$^A$.

In another example, a projection angiographic imaging system 270, is provided. The projection angiographic imaging system comprises one or more processors 110 configured to:

receive S110 volumetric angiographic image data comprising a volumetric image 120 of the vascular region;
generate S120 projection angiographic image data comprising one or more projection images 130$_{1..i}$ of the vascular region;
adapt S130 the volumetric image 120 using the one or more projection images 130$_{1..i}$ to provide an adapted volumetric image 120$^A$; and
output S140 an image representation 140 of the vascular region, the image representation being generated using the adapted volumetric image 120$^A$.

The above examples are to be understood as illustrative of the present disclosure, and not restrictive. Further examples are also contemplated. For instance, the examples described in relation to a system, may also be provided by the computer-implemented method, or by the computer program product, or by the computer-readable storage medium, or by the projection angiographic imaging system 270, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

**Claims**

1. A system (100) for providing an image representation of a vascular region, the system comprising one or more processors (110) configured to:

   receive (S110) volumetric angiographic image data comprising a volumetric image (120) of the vascular region;
   receive (S120) projection angiographic image data comprising one or more projection images ($130_{1..i}$) of the vascular region;
   adapt (S130) the volumetric image (120) using the one or more projection images ($130_{1..i}$) to provide an adapted volumetric image ($120^A$); and
   output (S140) an image representation (140) of the vascular region, the image representation being generated using the adapted volumetric image ($120^A$).

2. The system according to claim 1, wherein the one or more processors (110) are further configured to register the one or more projection images ($130_{1..i}$) to the volumetric image (120) prior to adapting (S130) the volumetric image (120).

3. The system according to claim 1 or claim 2, wherein the vascular region comprises one or more vessels ($150_{1..j}$), and wherein the adapting (S130) comprises adapting the one or more vessels in the volumetric image (120) to optimize a correspondence between the one or more vessels ($150_{1..j}$) in each of the one or more projection images ($130_{1..i}$) and a corresponding projection of the one or more vessels ($150^P_{1..j}$) in the volumetric image.

4. The system according to any previous claim, wherein the vascular region comprises one or more vessels ($150_{1..j}$), and wherein the adapting (S130) comprises one or more of:

   i) deforming a path of the one or more vessels ($150_{1..j}$) in the volumetric image (120) to optimize a correspondence between a path of the one or more vessels ($150_{1..j}$) in each of the one or more projection images ($130_{1..i}$) and a corresponding projection of the path of the one or more vessels ($150^P_{1..j}$) in the volumetric image;
   ii) extending the one or more vessels ($150_{1..j}$) in the volumetric image (120) to optimize a correspondence between the one or more vessels ($150_{1..j}$) in each of the one or more projection images ($130_{1..i}$) and a corresponding projection of the one or more vessels ($150^P_{1..j}$) in the volumetric image;
   iii) deforming a cross sectional shape (160) of the one or more vessels ($150_{1..j}$) in the volumetric image (120) to optimize a correspondence between a dimension (170i) of a cross section through the one or more vessels ($150_{1..j}$) in each of the one or more projection images ($130_{1..i}$) and a dimension ($170^P_i$) of a corresponding projection of the cross sectional shape of the one or more vessels in the volumetric image;
   iv) deforming a cross sectional shape (160) and/or adjusting an intensity (190) within the cross sectional shape (160) of the one or more vessels ($150_{1..j}$) in the volumetric image (120) to optimize a correspondence between an intensity profile ($180_1$) of a cross section through the one or more vessels ($150_{1..j}$) in each of the one or more projection images ($130_{1..i}$) and an intensity profile ($180^P_1$) of a corresponding projection of the intensity within the cross sectional shape of the one or more vessels in the volumetric image.

5. The system according to any previous claim, wherein the projection angiographic image data comprises a plurality of projection images, and wherein the projection images ($130_{1..i}$) form a temporal series of images representing the vascular region at different points in time; and
   wherein the one or more processors (110) are configured to perform the adapting (S130), and the outputting (S140), for each image in the temporal series such that the outputted image representation (140) is updated throughout the temporal series of images.

6. The system according to claim 5, wherein the outputting (S140) an image representation (140) comprises simultaneously outputting the image representation generated at each of a plurality of the points in time for comparing the image representations generated at the different points in time.

7. The system according to claim 5, wherein the temporal series of images represent the vascular region in one or more cardiac phases; and
wherein the adapting (S130), and the outputting (S140), are performed selectively for the images corresponding to each of one or more cardiac phases.

8. The system according to claim 1 or claim 2, wherein the projection angiographic image data comprises a plurality of projection images of the vascular region; and wherein the projection images $(130_{1..i})$ represent different perspectives $(\theta_{1..i})$ of the vascular region; and wherein the adapting (S130) the volumetric image (120) using the one or more projection images $(130_{1..i})$, comprises:

modelling the vascular region using the projection images $(130_{1..i})$ to provide a model of the vascular region; and combining the model of the vascular region with the volumetric image (120) to provide the adapted volumetric image ($120^A$).

9. The system according to claim 8, wherein the vascular region comprises one or more vessels $(150_{1..j})$, and wherein the combining the model of the vascular region with the volumetric image (120) comprises:

comparing the modelled vascular region with the volumetric image (120) to identify one or more one or more vessels in the modelled vascular region that are missing from the volumetric image (120) or poorly represented in the volumetric image; and
augmenting the volumetric image (120) with the one or more missing, or poorly represented, vessels to provide the adapted volumetric image ($120^A$).

10. The system according to any previous claim, wherein the vascular region comprises one or more vessels $(150_{1..j})$, and wherein the one or more processors (110) are further configured to:

calculate, from the adapted volumetric image ($120^A$), or from the image representation (140), a value of one or more vessel parameters (210) for the one or more vessels $(150_{1..j})$; and
output the calculated value of the one or more vessel parameters, or a value of one or more parameters derived therefrom.

11. The system according to any previous claim, wherein the vascular region comprises one or more vessels $(150_{1..j})$, and wherein the one or more processors (110) are further configured to:

i) determine, using the adapted volumetric image ($120^A$), or the image representation (140), a recommended position (230) for a stent in at least one of the one or more vessels $(150_{1..j})$; and
output an indication of the recommended position for the stent;
and/or
ii) calculate, using the adapted volumetric image ($120^A$), or the image representation (140), a value of a blood flow parameter for at least one of the one or more vessels $(150_{1..j})$; and
output the value of the blood flow parameter.

12. The system according to any previous claim, wherein the image representation (140) of the vascular region comprises one or more of:

i) a three-dimensional rendering of the adapted volumetric image ($120^A$);
ii) a projection of the adapted volumetric image ($120^A$);
iii) a side by side positioning of the adapted volumetric image ($120^A$), and at least one of the one or more projection images $(130_{1..i})$;
iv) an overlay image comprising the adapted volumetric image ($120^A$), and at least one of the one or more projection images $(130_{1..i})$;
v) a schematic image representing the adapted volumetric image ($120^A$).

13. The system according to any previous claim, wherein the volumetric image (120), and the one or more projection images, each have a spatial resolution; and

wherein the spatial resolution of the volumetric image (120) is relatively lower than the spatial resolution of the one or more projection images.

14. The system according to any previous claim, wherein the projection angiographic image data comprises a plurality of projection images ($130_{1..i}$) of the vascular region; and
wherein the projection images ($130_{1..i}$) represent different perspectives ($\theta_{1..i}$) of the vascular region.

15. The system according to any previous claim, wherein the volumetric image (120) of the vascular region is a pre-procedural volumetric image of the vascular region, or a peri-procedural volumetric image of the vascular region; and
wherein the one or more projection images of the vascular region are intra-procedural projection images of the vascular region.

FIG. 1

FIG. 2

$130_i , (\theta_i)$

$\theta_1$

250

$\theta_2$

$\theta_3$

$\theta_i$

120, $120^A$

$150_{1..j}$

$130_3 , (\theta_3)$

260

$150^P_{1..j}$

$130_2 , (\theta_2)$

$150_{1..j}$

$130_1 , (\theta_1)$

# FIG. 3

FIG. 4

FIG. 5

FIG. 6

Vessel cross sectional area (mm²)

Cross-sectional area of plaque deposit (mm²)

Vessel lumen cross sectional area (mm²)

210

140

# FIG. 7

210

Vessel diameter (mm)

Plaque  Bifurcation #1

Bifurcation #2

40°

80°

140

Vessel length (mm)

# FIG. 8

220

140

Lesion 1

Lesion 2

# FIG. 9

FIG. 10

**EUROPEAN SEARCH REPORT**

Application Number

EP 24 30 5095

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/037761 A1 (MISTRETTA CHARLES A [US] ET AL) 17 February 2011 (2011-02-17) | 1-7, 10-15 | INV. A61B6/50 G06T11/00 |
| Y | * paragraphs [0005;0029] - [0035;0043]; claim 19; figures 1-4 * | 8,9 | |
| X | EP 4 181 058 A1 (KONINKLIJKE PHILIPS NV [NL]) 17 May 2023 (2023-05-17) | 1-7,12, 14,15 | |
| Y | * paragraphs [0015;0018] - [0021;0033;0046]; figures 1-4 * | 8,9 | |
| Y | RIVEST-HENAULT D ET AL: "Nonrigid 2D/3D Registration of Coronary Artery Models With Live Fluoroscopy for Guidance of Cardiac Interventions", IEEE TRANSACTIONS ON MEDICAL IMAGING, IEEE, USA, vol. 31, no. 8, August 2012 (2012-08), pages 1557-1572, XP011491128, ISSN: 0278-0062, DOI: 10.1109/TMI.2012.2195009 * Section IV * | 8,9 | |
| A | MARKELJ P ET AL: "A review of 3D/2D registration methods for image-guided interventions", MEDICAL IMAGE ANALYSIS, OXFORDUNIVERSITY PRESS, OXFORD, GB, vol. 16, no. 3, April 2012 (2012-04), pages 642-661, XP002696430, ISSN: 1361-8423, DOI: 10.1016/J.MEDIA.2010.03.005 [retrieved on 2010-04-13] * the whole document * | 1-15 | TECHNICAL FIELDS SEARCHED (IPC) A61B G06T G06V |

The present search report has been drawn up for all claims

| Place of search | Date of completion of the search | Examiner |
|---|---|---|
| The Hague | 13 June 2024 | Craciun, Paula |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 30 5095

This annex lists the patent family members relating to the patent documents cited in the above-mentioned European search report.
The members are as contained in the European Patent Office EDP file on
The European Patent Office is in no way liable for these particulars which are merely given for the purpose of information.

13-06-2024

| Patent document cited in search report | Publication date | Patent family member(s) | Publication date |
|---|---|---|---|
| US 2011037761 A1 | 17-02-2011 | US 2011037761 A1<br>US 2014142423 A1 | 17-02-2011<br>22-05-2014 |
| EP 4181058 A1 | 17-05-2023 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• WO 2016001017 A1

**Non-patent literature cited in the description**

• **CIMEN, S. et al.** Reconstruction of coronary arteries from X-ray angiography: A review. *Med Image Anal*, August 2016, vol. 32, 46-68